(19) Europäisches Patentamt
European Patent Office
Office européen des brevets

(11) **EP 4 520 183 A1**

(12) **EUROPEAN PATENT APPLICATION**
published in accordance with Art. 153(4) EPC

(43) Date of publication:
**12.03.2025 Bulletin 2025/11**

(21) Application number: 23799483.5

(22) Date of filing: **28.04.2023**

(51) International Patent Classification (IPC):
*A23J 3/14* (2006.01)    *A23L 11/60* (2025.01)
*A23L 11/65* (2025.01)    *A23L 2/66* (2006.01)
*A23L 2/38* (2021.01)

(52) Cooperative Patent Classification (CPC):
A23J 3/14; A23L 2/38; A23L 2/66; A23L 11/60;
A23L 11/65

(86) International application number:
**PCT/JP2023/016982**

(87) International publication number:
**WO 2023/214553 (09.11.2023 Gazette 2023/45)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.05.2022 JP 2022076594**

(71) Applicant: **Amano Enzyme Inc.**
**Nagoya-shi**
**Aichi 460-8630 (JP)**

(72) Inventor: **SAKAI, Kiyota**
**Kakamigahara-shi, Gifu 509-0109 (JP)**

(74) Representative: **Müller-Boré & Partner**
**Patentanwälte PartG mbB**
**Friedenheimer Brücke 21**
**80639 München (DE)**

(54) **FOOD TEXTURE IMPROVER FOR VEGETABLE-PROTEIN-CONTAINING LIQUID COMPOSITION**

(57) The purpose of the present invention is to provide a processing technique for obtaining a vegetable-protein-containing liquid composition whose gritty texture is improved despite having a high vegetable protein material content of 6 wt% or greater. The food texture of a vegetable-protein-containing liquid composition having a vegetable protein material content of 6 wt% or greater can be improved by treating this vegetable-protein-containing liquid composition with a protein deamidase.

EP 4 520 183 A1

**Description**

Technical Field

The present invention relates to a food texture improver for a plant protein-including liquid composition. More specifically, the present invention relates to a processing technique for a plant protein-including liquid composition with improved gritty texture while having a high plant protein material content of 6 wt% or more.

Background Art

For various reasons such as a recent health boom, countermeasures against allergy problems, and religious reasons, plant milk rich in nutrients has become popular as an alternative to animal milk.

Unlike animal milk, which is generally a secretory fluid, plant milk is prepared so as to disperse a pulverized product of plant tissue in water, and thus, smoothness of mouth is required as a basic characteristic as an alternative to animal milk. It is desirable that this basic characteristic is also taken into consideration in the process of improving plant milk. For example, PTL 1 describes that, in order to improve soy milk to contain a high concentration of soybean isoflavone, soy milk, which is smooth and includes a high concentration of soybean isoflavone, can be produced by grinding a soybean embryo stem or a processed product of the soybean embryo stem having a total amount of soybean isoflavone of 1050 to 2800 mg/100 g with soy milk so as to contain 4 to 10 wt% of the soybean embryo stem or the processed product thereof, and wet-pulverizing the resultant product by a high-pressure homogenization treatment without separating okara.

Citation List

Patent Literature

PTL 1: WO 2005/087019 A

Summary of Invention

Technical Problem

Since plant milk has a smaller amount of protein than animal milk, there is a problem in terms of protein intake efficiency. When the present inventor tried to prepare plant milk having a high protein content, the present inventor faced a problem in that when a plant protein material has a high content of 6 wt% or more, the food texture is remarkably deteriorated to such an extent that gritty texture is generated.

Therefore, an object of the present invention is to provide a processing technique for a plant protein-including liquid composition with improved gritty texture while having a high plant protein material content of 6 wt% or more.

Solution to Problem

The present inventor has found that the gritty texture of a plant protein-including liquid composition to be obtained can be improved by treating a plant protein-including liquid composition having a plant protein material content of 6 wt% or more with a protein deamidase. The present inventors have conducted further studies based on the findings, leading to the completion of the present invention.

That is, the present invention provides inventions of the following aspects.

Item 1. A food texture improver including a protein deamidase, the food texture improver being used for improving a food texture of a plant protein-including liquid composition having a plant protein material content of 6 wt% or more.

Item 2. The food texture improver according to item 1, in which a plant protein content in the plant protein-including liquid composition is 5 wt% or more.

Item 3. The food texture improver according to item 1 or 2, in which the plant protein is a protein of a plant selected from the group consisting of soybean, pea, lentil bean, chickpea, fava bean, mung bean, lupine bean, wheat, barley, oat, sorghum, corn, peanut, hemp seed, and chia seed.

Item 4. The food texture improver according to any one of items 1 to 3, in which the plant protein is a protein of a plant selected from the group consisting of lupine bean, wheat, oat, corn, hemp seed, and chia seed.

Item 5. A method for producing a plant protein-including liquid composition with improved food texture, the method including a step of allowing a protein deamidase to act on a plant protein-including liquid composition having a plant protein material content of 6 wt% or more.

Advantageous Effects of Invention

According to the present invention, there is provided a processing technique for a plant protein-including liquid composition with improved gritty texture while having a high plant protein material content of 6 wt% or more.

Description of Embodiments

## 1. Food Texture Improver for Plant Protein-Including Liquid Composition

A food texture improver of the present invention includes a protein deamidase, and is used for improving a food texture of a plant protein-including liquid composition having a plant protein material content of 6 wt% or more. Hereinafter, the food texture improver of the present invention will be described in detail.

### 1-1. Use Application

The food texture improver of the present invention is used for improving a food texture of a plant protein-including liquid composition having a plant protein material content of 6 wt% or more. In the present invention, the food texture refers to a rough feeling to the tongue. The improvement in food texture means to reduce the degree of rough feeling to the tongue, and preferably to impart smoothness.

Note that, the improvement in food texture can be confirmed organoleptically by feeling with the tongue that the degree of gritty texture is reduced when a plant protein-including liquid composition treated with the food texture improver of the present invention is contained in the mouth, as compared with the case of a plant protein-including liquid composition not treated with the food texture improver of the present invention. The improvement in food texture can also confirmed by the fact that the coefficient of friction obtained by measuring the coefficient of friction of a plant protein-including liquid composition treated with the food texture improver of the present invention using an apparatus simulating the inside of an oral cavity with an artificial tongue and an artificial saliva is lower than that of a plant protein-including liquid composition not treated with the food texture improver of the present invention.

The plant protein-including liquid composition to which the food texture improver of the present invention is applied is not particularly limited as long as it is a liquid including a plant protein in water. Specific examples of the plant protein-including liquid composition include (i) a liquid obtained by dispersing, in water, dry powder of a food material including a plant protein (specifically, at least one of a plant organ of a plant protein-derived plant and a material obtained by, for example, removing at least a part of components other than the protein from the plant organ to increase the content of the protein is exemplified; the same applies hereinafter); (ii) a liquid obtained by crushing and dispersing a food material including a plant protein in water, and removing an insoluble matter derived from a skin or the like of the food material by any means such as centrifugation, filtration, filtration bag, or sieve, as necessary; (iii) a liquid obtained by, for example, removing components other than the plant protein from the liquid of the above (i) or (ii) to increase the content of the plant protein; and (iv) a liquid obtained by mixing dry powder prepared from the liquid of any one of the above (i) to (iii) with water. As a preferred examples of the plant protein-including liquid composition, plant milk is mentioned.

There is no particular restriction on the food material including a plant protein. Specific examples of the food material including a plant protein include beans such as soybean, pea, lentil bean, chickpea, black bean, fava bean, mung bean, lupine bean, and haricot bean; cereals such as wheat, barley, oat, sorghum, rice, rye, buckwheat, Japanese barnyard millet, foxtail millet, teff, corn, and potato; nuts such as almond, coconut, peanut, cashew nut, hazelnut, pecan nut, macadamia nut, pistachio, walnut, brazil nut, pili nut, chestnut, sesame, and pine nut; and seeds such as hemp seed (the term "hemp" refers to industrial hemp, and specifically refers to a cultivar which does not contain tetrahydrocannabinol (THC) causing perceptual alterations or has a low THC concentration; since industrial hemp does not contain THC or has a low concentration thereof, it has no efficacy as a perceptual alteration drug or cannot lead to abuse), chia seed, quinoa, Amaranthus, canary seed, and linseed. The plant protein-including liquid composition may contain the above-described plant protein alone or a combination of two or more kinds thereof. Among these plant proteins, from the viewpoint of further improving the effect of improving the food texture, soybean, pea, lentil bean, chickpea, fava bean, mung bean, lupine bean, wheat, barley, oat, sorghum, corn, peanut, hemp seed, and chia seed are preferable, and lupine bean, wheat, oat, corn, hemp seed, and chia seed are more preferable. Note that, the plant protein included in the liquids of the above (i) to (iv) may be in any forms such as a protein (natural protein) included in a plant organ; a chemically partially decomposed protein of the above-described protein by an acid, an alkali, or the like; an enzymatically partially decomposed protein by a protease or the like; and a chemically modified protein by various reagents.

Note that, in the present invention, the "plant protein material content" in the plant protein-including liquid composition is a proportion occupied by the dry weight of the component constituting the above-described plant organ included in the plant protein-including liquid composition. For example, when the plant protein-including liquid composition is a liquid composed only of a component derived from a plant organ and water as in the liquids of the above (i) to (iv), the "plant protein material content" refers to a proportion occupied by the dry weight of the liquid. For example, when the plant protein-including liquid composition is a liquid including the liquids of the above (i) to (iv) and an additive, the "plant protein material content" refers to a proportion occupied by the dry weight of a portion obtained by removing the additive from the liquid.

The plant protein material content in the plant protein-including liquid composition to which the food texture improver of the present invention is applied is 6 wt% or more. When the plant protein material content of the plant protein-including liquid composition is as high as 6 wt% or more, the food texture remarkably decreases to such an extent that gritty texture is generated, but according to the food texture improver of the present invention, the gritty texture can be effectively improved even in a plant protein-including liquid composition having a high plant protein material content. Therefore, as a suitable example of the plant protein material content of the plant protein-including liquid composition, the content is preferably 7 wt% or more, more preferably 8 wt% or more, and further preferably 9 wt% or more.

The upper limit of the plant protein material content in the plant protein-including liquid composition is not particularly limited, and is, for example, 20 wt% or less. From the viewpoint of further improving the effect of improving the gritty texture, the upper limit of the plant protein material content in the plant protein-including liquid composition is preferably 17 wt% or less, more preferably 13 wt% or less, and further preferably 11 wt% or less.

The plant protein content in the plant protein-including liquid composition is determined according to the type of a food material serving as a plant protein source and the content of the plant protein material, and is, for example, 1 wt% or more, preferably 5 wt% or more, more preferably 6 wt% or more, further preferably 6.5 wt% or more, even more preferably 7 wt% or more or 7.5 wt% or more, and further more preferably 8 wt% or more, and the upper limit thereof is, for example, 20 wt% or less, preferably 17 wt% or less, more preferably 13 wt% or less, further preferably 11 wt% or less, and even more preferably 10 wt% or less.

## 1-2. Protein Deamidase

The food texture improver of the present invention includes a protein deamidase as an active ingredient. The food texture improver of the present invention can improve the gritty texture of the plant protein-including liquid composition to be obtained, by action of the protein deamidase on the plant protein-including liquid composition at a predetermined concentration.

The protein deamidase used in the present invention is an enzyme that exhibits an action of decomposing an amide group-including side chain of a protein without cleaving peptide bonds and without crosslinking the protein, and the type, origin, and the like thereof are not particularly limited. As long as the above action is main activity, the protein deamidase may further has an action of decomposing an amide group-including side chain of a protein with cleaving peptide bonds and crosslinking the protein.

Examples of the protein deamidase include an enzyme that deamidates a glutamine residue in a protein and converts it into glutamic acid (for example, protein glutaminase), an enzyme that deamidates an asparagine residue in a protein and converts it into aspartic acid (for example, protein asparaginase), and an enzyme that deiminizes a guanidino group of an arginine residue at the C-terminal of a protein (for example, protein arginine deiminase). Among them, from the viewpoint of further improving the effect of improving the food texture, protein glutaminase is preferable.

More specific examples of the protein deamidase include a protein deamidase derived from the genus Chryseobacterium, Flavobacterium, Empedobacter, Sphingobacterium, Aureobacterium, Myroides, Luteimicrobium, Agromyces, Microbacterium, or Leifsonia. These protein deamidases are known, and for example, JP2000-50887A, JP2001-218590A, WO2006/075772A1, WO2015/133590, and the like can be referred to. These protein deamidases may be used singly or in combination of a plurality of kinds thereof.

Among these protein deamidases, from the viewpoint of further improving the effect of improving the food texture, a protein deamidase derived from the genus Chryseobacterium is preferable, a protein glutaminase derived from the genus Chryseobacterium is more preferable, a protein glutaminase derived from Chryseobacterium proteolyticum sp. is further preferable, and a protein glutaminase derived from Chryseobacterium proteolyticum strain 9670 is even more preferable.

The protein deamidase can be prepared from a culture solution of a microorganism from which the protein deamidase is derived. Specific examples of the preparation method include a method of recovering a protein deamidase from a culture solution or a bacterial cell of the above-mentioned microorganism. For example, in the case of using a microorganism that secrets a protein deamidase, an enzyme can be separated and/or purified after recovering bacterial cells from the culture solution in advance by filtration, a centrifugal treatment, or the like, as necessary. In the case of using a microorganism that does not secret a protein deamidase , an enzyme can be separated and/or purified after recovering bacterial cells from the culture solution in advance as necessary and then disrupting the bacterial cells by a pressurization treatment, an ultrasonic treatment, a lytic enzymatic treatment, or the like to expose an enzyme. As an enzyme separation and/or purification method, a known protein separation and/or purification method can be used without particular limitation, and examples thereof include a centrifugal separation method, a UF concentration method, a salting-out method, and various chromatography methods using an ion-exchange resin or the like. The separated and/or purified enzyme can be powdered by a drying method such as freeze-drying or reduced-pressure drying, and can also be powdered using an appropriate excipient and/or drying aid in the drying method. The separated and/or purified enzyme can also be liquefied by adding an appropriate additive and performing filtration sterilization.

The amount of the protein deamidase included in the food texture improver of the present invention is not particularly limited as long as the effect of the protein deamidase can be effectively obtained when the food texture improver of the present invention is used, and is, for example, 0.1 to 5,000 U/g.

Note that, for the activity of the protein deamidase, benzyloxycarbonyl-L-glutaminylglycine (Z-Gln-Gly) is used as a substrate, and the amount of enzyme that liberates 1 $\mu$mol of ammonia per minute is defined as 1 unit (1 U).

1-3. Other components

The food texture improver of the present invention may or may not contain other components to the extent that the effect of the present invention is not affected, in addition to the protein deamidase described above. Examples of the other components include enzymes other than the protein deamidase and additives.

As other enzymes, one or more enzymes can be appropriately selected according to a desired reaction other than the reaction by the protein deamidase based on the type or composition of the plant protein-including liquid composition to which the food texture improver of the present invention is applied.

The additive can be appropriately selected according to a desired formulation form. The formulation form of the food texture improver of the present invention is not particularly limited, and may be either a solid formulation or a liquid formulation. Therefore, examples of the additive include an excipient, a buffer, a suspending agent, a stabilizer, a preservative, an antiseptic agent, water, and a pH adjuster. These additives may be included singly or in combination of a plurality of kinds thereof.

2. Method for Producing Plant Protein-Including Liquid Composition with Improved Food Texture

The present invention also provides a method for producing a plant protein-including liquid composition with improved food texture, the method including a step of allowing a protein deamidase to act on a plant protein-including liquid composition having a plant protein material content of 6 wt% or more.

2-1. Step of Allowing Protein Deamidase to Act

The type of the plant protein-including liquid composition, the type of the protein deamidase, and the like in the step of allowing a protein deamidase to act are as shown in the section "1. Food Texture Improver for Plant Protein-Including Liquid Composition" described above.

Regarding the amount of the protein deamidase used, the amount of the protein deamidase used per 1 g (dry weight) of the plant protein material included in the plant protein-including liquid composition is, for example, 0.25 U or more and preferably 0.5 U or more. From the viewpoint of further improving the effect of improving the food texture, the amount of the protein deamidase used per 1 g (dry weight) of the plant protein material included in the plant protein-including liquid composition is preferably 1 U or more, more preferably 2 U or more, further preferably 3 U or more, and even more preferably 4 U or more or 4.5 U or more. The upper limit of the range of the amount of the protein deamidase used per 1 g (dry weight) of the plant protein material included in the plant protein-including liquid composition is not particularly limited, and is, for example, 500 U or less, 250 U or less, 100 U or less, 75 U or less, 50 U or less, 25 U or less, 10 U or less, or 7.5 U or less.

Regarding the amount of the protein deamidase used, the amount of the protein deamidase used per 1 g of the plant protein included in the plant protein-including liquid composition is, for example, 0.25 U or more and preferably 0.5 U or more. From the viewpoint of further improving the effect of improving the food texture, the amount of the protein deamidase used per 1 g of the plant protein included in the plant protein-including liquid composition is preferably 1 U or more, more preferably 2 U or more, further preferably 3 U or more, and even more preferably 4 U or more or 4.5 U or more. The upper limit of the range of the amount of the protein deamidase used per 1 g of the plant protein included in the plant protein-including liquid composition is not particularly limited, and is, for example, 500 U or less, 250 U or less, 100 U or less, 75 U or less, 50 U or less, 25 U or less, or 20 U or less.

In the step of allowing a protein deamidase to act, the plant protein-including liquid composition and the protein deamidase are mixed, and the protein deamidase is allowed to act on the protein in the plant protein-including liquid composition, thereby allowing a reaction for improving the food texture to proceed. As for the reaction conditions, the reaction temperature and pH at which the protein deamidase acts on the protein in the plant protein-including liquid composition, the reaction time for allowing the reaction to proceed sufficient to improve the food texture, and the like can be appropriately set by those skilled in the art through a preliminary test and the like in consideration of the temperature and pH characteristics of the protein deamidase to be used.

The reaction temperature can be determined according to the optimum temperature of the protein deamidase to be used, and the like, and is, for example, 10 to 80°C, preferably 20 to 70°C, more preferably 40 to 60°C, and further preferably 45 to 55°C. The pH can be determined according to the optimum pH of the protein deamidase to be used, and the like, and the pH at 25°C is, for example, 5 to 10, preferably 5 to 8, and more preferably 6 to 8. The reaction time can be determined according to a reaction scale and the like, and is, for example, 1 to 48 hours, preferably 1 to 24 hours, and more preferably 8 to 20 hours or 10 to 18 hours.

## 2-2. Other Steps

The production method of the present invention can include other steps in addition to the above-described step of allowing a protein deamidase to act.
Specific examples of the other steps include a deactivation step of a protein deamidase, a cooling step, and a filtration step.

## 2-3. Plant Protein-Including Liquid Composition with Improved Food Texture

The plant protein-including liquid composition with improved food texture obtained by the production method of the present invention is imparted with improved food texture as compared with a plant protein-including liquid composition not treated with the food texture improver of the present invention. The obtained plant protein-including liquid composition with improved food texture can be used, for example, as a plant alternative milk, an alternative cream, an alternative yogurt, an alternative cheese, an alternative ice cream, or the like as it is or by being appropriately prepared. Examples
Hereinafter, the present invention will be specifically described by means of Examples; however, the present invention is not to be construed as being limited to the following Examples.

[Used Enzyme]

As the protein deamidase, PG which is a protein glutaminase derived from Chryseobacterium proteolyticum (manufactured by Amano Enzyme Inc.) was used.

[Enzyme Activity Measurement Method]

To 1 mL of a 0.2 M phosphate buffer (pH 6.5) including 30 mM Z-Gln-Gly, 0.1 mL of a sample solution including a protein deamidase was added, the mixture was left to stand at 37°C for 10 minutes, and then 1 mL of a 0.4 M TCA solution was added to stop the reaction. As a blank, to 1 mL of a 0.2 M phosphate buffer (pH 6.5) including 30 mM Z-Gln-Gly, 1 mL of a 0.4 M TCA solution was added, 0.1 mL of a sample solution including a protein deamidase was further added, and the mixture was left to stand at 37°C for 10 minutes.
The amount of ammonia generated in the reaction solution was measured for the solution obtained above using Ammonia Test Wako (FUJIFILM Wako Pure Chemical Corporation). The ammonia concentration in the reaction solution was determined from a calibration curve representing the relationship between the ammonia concentration and the absorbance (630 nm) prepared using an ammonia standard solution (ammonium chloride).
The activity of the protein deamidase was calculated from the following formula with the amount of enzyme that produces 1 $\mu$mol of ammonia per minute being defined as 1 unit (1 U). In the formula, the reaction solution amount is 2.1, the enzyme solution amount is 0.1, and Df is a dilution rate of the enzyme solution. 17.03 is a molecular weight of ammonia.

[Mathematical Formula 1]

Protein deamidase activity (U/mL)
= Ammonia concentration (mg/L) in reaction solution $\times$ (1/17.03)
$\times$ (Reaction solution amount/Enzyme solution amount) $\times$ (1/10) $\times$ Df

[Food Material Serving as Plant Protein Source (Hereinafter, "Plant Protein Material")]

Plant protein materials (all powders) shown in the following table were used.

[Table 1]

| Product name | Plant protein | Protein content (weight basis) | Manufacturer |
|---|---|---|---|
| SOYPRO | Soybean protein | 50% or more | J-OIL MILLS, INC. |
| NUTRALYS F85M | Pea protein | 85% or more | Roquette Japan K.K. |
| VITEN | Wheat protein | 80% or more | Roquette Japan K.K. |
| MUNG BEAN PROTEIN | Mung bean protein | 80% or more | Organo Corporation |
| FAVA BEAN PROTEIN | Fava bean protein | 83.7% or more | Organo Corporation |
| CHICKPEA PROTEIN | Chickpea protein | 84% or more | Organo Corporation |
| OAT PROTEIN | Oat protein | 75% or more | Organo Corporation |
| CHIA SEED PROTEIN | Chia seed protein | 83% or more | Organo Corporation |
| LENTIL BEAN PROTEIN | Lentil bean protein | 50% or more | Organo Corporation |
| PEANUT PROTEIN | Peanut protein | 26.5% or more | Morimoto Shoten |
| HEMP PROTEIN | Hemp seed(*) protein | 85% or more | Bio Actives Japan Corporation |
| ZEIN | Corn protein | 81% or more | FUJIFILM Wako Pure Chemical Corporation |
| Lupin isolate | Lupine bean protein | 85% or more | Wide Open Agriculture |
| ISHIBASHIKOHGYO BARLEY POWDER | Barley protein | 6.8% | Ishibashikohgyo Co., Ltd. |
| WHITE SORGHUM FLOUR | Sorghum protein | 10% | NAKANO INDUSTRY CO., LTD. |
| (*) Hemp seed is derived from industrial hemp not including THC. | | | |

[Test Example]

(1) Test Operation

Each plant protein material in Table 1 was mixed with a phosphate buffer having a pH of 7.0 (25°C) so that the plant protein material had a content shown in Tables 2 to 5, thereby preparing a plant protein-including liquid composition. To the plant protein-including liquid composition, PG in an amount of 5 U per 1 g of the plant protein material was added, and the mixture was incubated at 50°C overnight to obtain a treated plant protein-including liquid composition (PG "Present"). Separately, the same operation was performed except that PG was not added to obtain a treated plant protein-including liquid composition (PG "Absent").

(2) Evaluation

(2-1) Food Texture Evaluation

The treated plant protein-including liquid composition obtained in the above (1) was contained in the mouth of each of two trained panelists, and the food texture (feeling on the tongue) was checked and classified based on the following criteria. The results are shown in Tables 2 to 5.

×× : Flakiness is strongly felt.
x: Flakiness is felt.
Δ: Flakiness almost disappears.
∘: Smoothness is felt.
⊙: Smoothness is strongly felt.

(2-2) Protein Solubility Evaluation

The treated plant protein-including liquid composition obtained in the above (1) was centrifuged at 15,000 g for 5 minutes. The supernatant was collected, and the protein concentration was quantified using Bradford reagent manufactured by Bio-Rad Laboratories, Inc. A relative value of the protein concentration of the treated plant protein-including liquid composition (PG "Present") was derived when the protein concentration of the treated plant protein-including liquid composition (PG "Absent") was taken as "1". The results are shown in Tables 2 to 5.

[Table 2]

| | Reference Example 1 | Reference Example 2 | Comparative Example 1 | Example 1 | Comparative Example 2 | Example 2 | Reference Example 3 | Reference Example 4 | Comparative Example 3 | Example 3 | Comparative Example 4 | Example 4 |
|---|---|---|---|---|---|---|---|---|---|---|---|---|
| Type | Oat | | Oat | | Oat | | Soybean | | Soybean | | Soybean | |
| Plant protein material content | 0.2 wt% | | 6 wt% | | 10 wt% | | 3 wt% | | 6 wt% | | 10 wt% | |
| Plant protein content | 0.15 to 0.2 wt% | | 4.5 to 6 wt% | | 7.5 to 10 wt% | | 1.5 to 3 wt% | | 3 to 6 wt% | | 5 to 10 wt% | |
| PG | Absent | Present | Absent | Present | Absent | Present | Absent | Present | Absent | Present | Absent | Present |
| Food texture | ○ | ⊙ | xx | ⊙ | xx | ⊙ | ○ | ○ | xx | ○ | xx | ○ |
| Degree of solubility of protein (relative value) | | | | | 1 | 7.7 | | | | | 1 | 3.3 |

[Table 3]

| | Comparative Example 5 | Example 5 | Comparative Example 6 | Example 6 | Comparative Example 7 | Example 7 | Comparative Example 8 | Example 8 | Comparative Example 9 | Example 9 |
|---|---|---|---|---|---|---|---|---|---|---|
| Type | Lupine bean | | Chia seed | | Corn | | Hemp seed | | Wheat | |
| Plant protein material content | 10 wt% | | 10 wt% | | 10 wt% | | 10 wt% | | 10 wt% | |
| Plant protein content | 8.5 to 10 wt% | | 8.3 to 10 wt% | | 8.1 to 10 wt% | | 8.5 to 10 wt% | | 8 to 10 wt% | |
| PG | Absent | Present | Absent | Present | Absent | Present | Absent | Present | Absent | Present |
| Food texture | xx | ⊙ | xx | ⊙ | xx | ⊙ | xx | ⊙ | xx | ⊙ |
| Degree of solubility of protein (relative value) | 1 | 7.3 | 1 | 6.3 | 1 | 13.7 | 1 | 12.1 | 1 | 12.2 |

[Table 4]

| | Comparative Example 10 | Example 10 | Comparative Example 11 | Example 11 | Comparative Example 12 | Example 12 | Comparative Example 13 | Example 13 | Comparative Example 14 | Example 14 | Comparative Example 15 | Example 15 |
|---|---|---|---|---|---|---|---|---|---|---|---|---|
| Type | Fava bean | | Mung bean | | Chickpea | | Lentil bean | | Sorghum | | Peanut | |
| Plant protein material content | 10 wt% | | 10 wt% | | 10 wt% | | 10 wt% | | 10 wt% | | 10 wt% | |
| Plant protein content | 8.37 to 10 wt% | | 8 to 10 wt% | | 8.4 to 10 wt% | | 5 to 10 wt% | | 1 wt% | | 2.65 to 10 wt% | |
| PG | Absent | Present | Absent | Present | Absent | Present | Absent | Present | Absent | Present | Absent | Present |
| Food texture | xx | ○ | xx | ○ | ×× | ○ | xx | ○ | xx | ○ | xx | ○ |
| Degree of solubility of protein (relative value) | 1 | 5.4 | 1 | 6.5 | 1 | 5.3 | 1 | 5.9 | 1 | 4.6 | 1 | 0.9 |

[Table 5]

| | Comparative Example 16 | Example 16 | Comparative Example 17 | Example 17 |
|---|---|---|---|---|
| Type | Pea | | Barley | |
| Plant protein material content | 10 wt% | | 10 wt% | |
| Plant protein content | 8.5 to 10 wt% | | 1 wt% | |
| PG | Absent | Present | Absent | Present |
| Food texture | × | ○ | × | ○ |
| Degree of solubility of protein (relative value) | 1 | 4.3 | 1 | 1.7 |

As shown in Reference Examples 1 and 3, the plant protein-including liquid composition having a low content of the plant protein material did not cause a problem of gritty texture, but as shown in Comparative Examples 1 to 17, the plant protein-including liquid composition having a high content of the plant protein material remarkably caused a problem of gritty texture. As shown in Reference Examples 2 and 4, in the case of subjecting the plant protein-including liquid composition having a low content of the plant protein material to PG treatment, the food texture was slightly improved or a change in food texture was not observed, but as shown in Examples 1 to 17, in the case of subjecting the plant protein-including liquid composition having a high content of the plant protein material to PG treatment, the food texture was remarkably improved. In particular, when the plant protein material is oat, lupine bean, wheat, corn, hemp seed, or chia seed, the effect of improving the food texture was especially remarkable.

**Claims**

1.   A food texture improver comprising a protein deamidase, the food texture improver being used for improving a food texture of a plant protein-including liquid composition having a plant protein material content of 6 wt% or more.

2.   The food texture improver according to claim 1, wherein a plant protein content in the plant protein-including liquid composition is 5 wt% or more.

3.   The food texture improver according to claim 1, wherein the plant protein is a protein of a plant selected from the group consisting of soybean, pea, lentil bean, chickpea, fava bean, mung bean, lupine bean, wheat, barley, oat, sorghum, corn, peanut, hemp seed, and chia seed.

4.   The food texture improver according to claim 1, wherein the plant protein is a protein of a plant selected from the group consisting of lupine bean, wheat, oat, corn, hemp seed, and chia seed.

5.   A method for producing a plant protein-including liquid composition with improved food texture, the method comprising a step of allowing a protein deamidase to act on a plant protein-including liquid composition having a plant protein material content of 6 wt% or more.

# INTERNATIONAL SEARCH REPORT

| International application No. |
|---|
| **PCT/JP2023/016982** |

## A. CLASSIFICATION OF SUBJECT MATTER

*A23J 3/14*(2006.01)i; *A23L 11/60*(2021.01)i; *A23L 11/65*(2021.01)i; *A23L 2/66*(2006.01)i; *A23L 2/38*(2021.01)i
FI:   A23J3/14; A23L2/00 J; A23L2/38 D; A23L2/38 C; A23L11/65; A23L11/60

According to International Patent Classification (IPC) or to both national classification and IPC

## B. FIELDS SEARCHED

Minimum documentation searched (classification system followed by classification symbols)

A23J3/14; A23L11/60; A23L11/65; A23L2/66; A23L2/38

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| X | WO 2022/092241 A1 (AMANO ENZYME INC.) 05 May 2022 (2022-05-05) claims, paragraphs [0012]-[0020], examples | 1-5 |
| X | WO 2022/071418 A1 (AMANO ENZYME INC.) 07 April 2022 (2022-04-07) claims, paragraphs [0015]-[0020], examples | 1-5 |
| X | WO 2021/251344 A1 (AMANO ENZYME INC.) 16 December 2021 (2021-12-16) claims, paragraph [0019], examples | 1-5 |
| X | WO 2021/049591 A1 (AMANO ENZYME INC.) 18 March 2021 (2021-03-18) claims, examples | 1-5 |
| X | WO 2022/045152 A1 (AMANO ENZYME INC.) 03 March 2022 (2022-03-03) claims, examples | 1-5 |
| X | WO 2020/171106 A1 (AMANO ENZYME INC.) 27 August 2020 (2020-08-27) claims, examples | 1-5 |
| P, X | WO 2022/097675 A1 (AMANO ENZYME INC.) 12 May 2022 (2022-05-12) claims, examples | 1-5 |

☑ Further documents are listed in the continuation of Box C.　　☑ See patent family annex.

| | |
|---|---|
| *　Special categories of cited documents: | "T"　later document published after the international filing date or priority date and not in conflict with the application but cited to understand the principle or theory underlying the invention |
| "A"　document defining the general state of the art which is not considered to be of particular relevance | |
| "E"　earlier application or patent but published on or after the international filing date | "X"　document of particular relevance; the claimed invention cannot be considered novel or cannot be considered to involve an inventive step when the document is taken alone |
| "L"　document which may throw doubts on priority claim(s) or which is cited to establish the publication date of another citation or other special reason (as specified) | "Y"　document of particular relevance; the claimed invention cannot be considered to involve an inventive step when the document is combined with one or more other such documents, such combination being obvious to a person skilled in the art |
| "O"　document referring to an oral disclosure, use, exhibition or other means | |
| "P"　document published prior to the international filing date but later than the priority date claimed | "&"　document member of the same patent family |

| Date of the actual completion of the international search | Date of mailing of the international search report |
|---|---|
| **05 June 2023** | **18 July 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2023/016982** |

| C. | DOCUMENTS CONSIDERED TO BE RELEVANT | |
| --- | --- | --- |
| Category* | Citation of document, with indication, where appropriate, of the relevant passages | Relevant to claim No. |
| P, X | WO 2022/102723 A1 (AMANO ENZYME INC.) 19 May 2022 (2022-05-19)<br>claims, examples | 1-5 |
| P, X | WO 2022/138872 A1 (AMANO ENZYME INC.) 30 June 2022 (2022-06-30)<br>claims, examples | 1, 2, 5 |

Form PCT/ISA/210 (second sheet) (January 2015)

EP 4 520 183 A1

## INTERNATIONAL SEARCH REPORT
### Information on patent family members

International application No.

**PCT/JP2023/016982**

| Patent document cited in search report | | | Publication date (day/month/year) | Patent family member(s) | | | Publication date (day/month/year) |
|---|---|---|---|---|---|---|---|
| WO | 2022/092241 | A1 | 05 May 2022 | CN 114521592 A claims, examples | | | |
| WO | 2022/071418 | A1 | 07 April 2022 | CN 114304276 A claims, examples | | | |
| WO | 2021/251344 | A1 | 16 December 2021 | (Family: none) | | | |
| WO | 2021/049591 | A1 | 18 March 2021 | US 2022/0338503 A1 claims, examples EP 4029947 A1 | | | |
| WO | 2022/045152 | A1 | 03 March 2022 | (Family: none) | | | |
| WO | 2020/171106 | A1 | 27 August 2020 | US 2022/0151255 A1 claims, examples US 2022/0151254 A1 EP 3928628 A1 EP 3928627 A1 WO 2020/171105 A1 | | | |
| WO | 2022/097675 | A1 | 12 May 2022 | CN 114521593 A claims, examples | | | |
| WO | 2022/102723 | A1 | 19 May 2022 | (Family: none) | | | |
| WO | 2022/138872 | A1 | 30 June 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005087019 A
- JP 2000050887 A
- JP 2001218590 A
- WO 2006075772 A1
- WO 2015133590 A